# EUROPEAN PATENT SPECIFICATION

(11) **EP 0 379 904 B1**
(45) Date of publication and mention of the grant of the patent: **08.05.1996**
(21) Application number: 90100557.9
(22) Date of filing: 12.01.1990
(51) Int. Cl.: C12N 15/12, C07K 14/435, C12P 21/02, C12P 21/08, C12N 5/10, A61K 38/17, A61K 39/395

(54) **A soluble molecule related to but distinct from ICAM-1**
Lösliches Molekül, das mit ICAM-1 verwandt, aber davon verschieden ist
Molécule souple apparentée à mais différente d'ICAM-1

(30) Priority: 24.01.1989 US 301192; 13.12.1989 US 445951
(43) Date of publication of application: 01.08.1990
(73) Proprietor: Bayer Corporation, Pittsburgh, PA 15219-2502 (US)
(72) Inventor: Greve, Jeffrey, Branford, CT 06405 (US); McClelland, Alan, Old Saybrook CT 06475 (US)
(74) Representative: Dänner, Klaus

(56) References cited:
- EP-A- 0 289 949
- EP-A- 0 314 863
- NATURE, vol. 331, no. 6157, February 1988, London D. SIMMONS et al. "ICAM, an adhesion ligand of LFA-1, is homologous to the neural cell adhesion molecule NCAM" pages 624-627
- CELL, vol. 52, no. 6, March 25, 1988, Cambridge, Mass., USA D.E. STAUNTON et al.
- Nature 344 (1990) 70-72

## Description

### BACKGROUND OF THE INVENTION

The present invention relates to a soluble form of intercellular adhesion molecule (sICAM-1) as well as the DNA sequence encoding sICAM-1. sICAM-1 and ICAM-1 have substantial similarity, in that they share the first 442 NH₂-terminal amino acids of the extracellular domain. However, sICAM-1 differs from ICAM-1 at the C-terminus, and these changes confer solubility to sICAM-1. ICAM-1 is known to mediate adhesion of many cell types, including endothelial cells, to lymphocytes which express lymphocyte function-associated antigen-1 (LFA-1). ICAM-1 has the property of directly binding LFA-1. There is also evidence for LFA-1 mediated adhesion which is not via ICAM-1. Additionally, ICAM-1 has the ability to bind both LFA-1 and human rhinovirus. It has the property of inhibiting infection of rhinovirus and Coxsackie A viruses. It may be used to antagonize adhesion of cells mediated by ICAM-1 binding including ICAM-1/LFA-1 binding and thus be useful in treatment of inflammation, graft rejection, LFA-1 expressing tumors, and other processes involving cell adhesion. Based on the substantial similarity of the extracellular domains of ICAM-1 and sICAM-1, sICAM-1 has the properties identified for ICAM-1.

The major Human Rhinovirus Receptor (HRR) has been transfected, identified, purified and reconstituted as described in co-pending European Patent Applications EP-A2-0 319 815 and EP-A1-0 362 531

This receptor has been shown to be identical to a previously described cell surface protein, ICAM-1. European Patent Applications 0 289 949 and 0 365 837 describe a membrane associated cell adhesion molecule (ICAM-1) which mediates attachment of many cell types including endothelial cells to lymphocytes which contain LFA-1. These patent applications provide a discussion of the present research in the field of intercellular adhesion molecules. It is important to note that the inventors specifically looked for an alternatively spliced mRNA for ICAM-1 and did not identify one. ICAM-1 was first identified based on its role in adhesion of leukocytes to T-cells (Rothlein, R. et al, J. Immunol. 137: 1270-1274 (1986)) which has been shown to be mediated by the heterotypic binding of ICAM-1 to LFA-1 (Marlin et al, Cell 51 :813-819 (1987)). The primary structure of ICAM-1 has revealed that it is homologous to the cellular adhesion molecules Neural Cell Adhesion Molecule (NCAM) and Mylein-Associated Glycoprotein (MAG), and has led to the proposal that it is a member of the immunoglobulin supergene family (Simmons et al, Nature 331: 624-627 (1988); Staunton et al, Cell 52: 925-933 (1988) The DNA sequence of cDNA clones are described in the above referenced papers by Simmons et al and Staunton et al, supra, from which the amino acid sequence of ICAM-1 can be deduced. The ICAM-1 molecule has a typical hydrophobic membrane spanning region containing 24 amino acids and a short cytoplasmic tail containing 28 amino acids. The ICAM-1 of the prior art is an insoluble molecule which is solubilized from cell membranes by lysing the cells in a non-ionic detergent. The solubilized ICAM-1 mixture in detergent is then passed through a column matrix material and then through a monoclonal antibody column matrix for purification.

### SUMMARY OF THE INVENTION

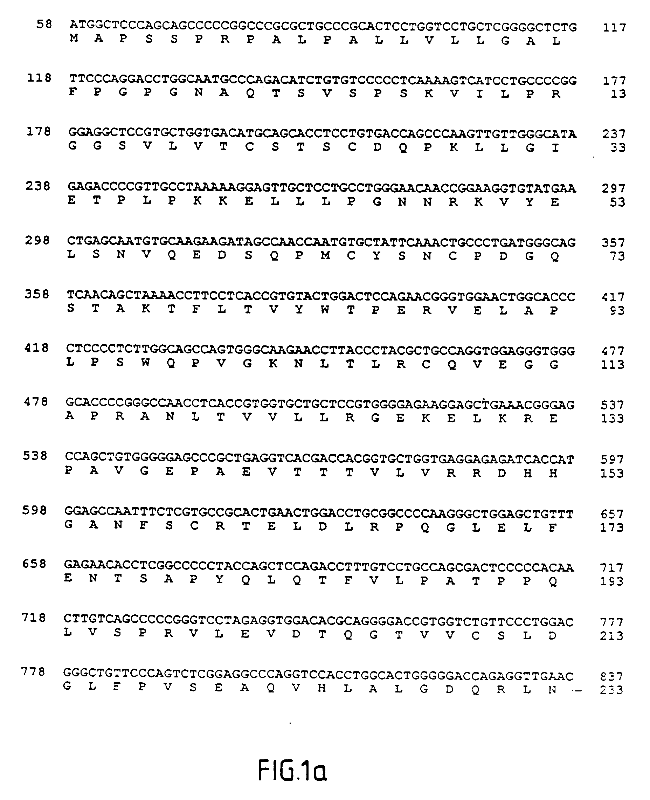

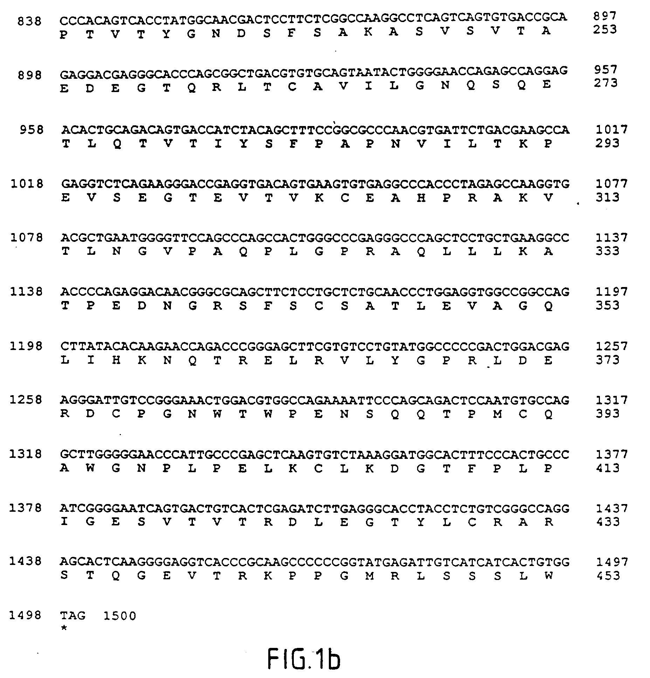

The present invention provides an endogenous alternatively spliced molecular species of ICAM-1 designated sICAM-1 which displays an alternative mRNA sequence and which is soluble without the addition of a detergent (see Figure 1).

The present invention provides purified and isolated human soluble intercellular adhesion molecule (sICAM-1) according to the sequence disclosed in Figure 1, or a functional derivative thereof with the provisio that the 11 C-terminal amino acids disclosed in Figure 1 remain unchanged, substantially free of natural contaminants. sICAM-1 can be obtained from HeLa, HE1 and primary transfectant cells thereof characterized by being soluble in the absence of detergents and being the translation product of the mRNA sequences as shown in Figure 1. This natural product of human cells has the advantage of being secreted from cells in a soluble form and not being immunogenic. The natural soluble product differs from the natural insoluble product in that the soluble product contains a novel sequence of 11 amino acid residues at the C-terminus and does not contain the membrane spanning and cytoplasmic domains present in the insoluble form.

The present invention provides a purified and isolated DNA sequence encoding sICAM-1 according to the sequence disclosed in Figure 1 as well as a host cell encoding said sequence.

The present invention provides a method of recovering soluble intercellular adhesion molecule in substantially pure form comprising the steps of:
(a) removing the supernatant from unlysed cells,
(b) introducing the supernatant to an affinity matrix containing immobilized antibody capable of binding to sICAM-1, preferably antibodies against ICAM-1 and sICAM-1, or derivatives thereof,
(c) permitting said sICAM-1 to bind to said antibody of said matrix,
(d) washing said affinity matrix to remove unbound contaminants, and
(e) recovering said sICAM-1 in substantially pure form by eluting said sICAM-1 from said matrix.

Further purificaton utilizing a lectin or wheat germ agglutinin column may be used before or after the antibody matrix step. Other purification steps could include sizing chromotography, ion chromotography, and gel electrophoresis. Further purification by velocity sedimentation through sucrose gradients may be used. The antibody capable of binding to sICAM-1 could include antibodies against ICAM-1 or HRR.

The present invention includes polyclonal and/or monoclonal antibodies against sICAM-1.

The present invention further includes an antibody preferably monoclonal specific for sICAM-1, capable of binding to the sICAM-1 molecule according to claim 1 and that is not capable of binding to ICAM-1 preferably capable of binding to the 11 amino acid sequence C terminus as shown in Figure 1. For a method for producing a peptide antisera see Green et al, Cell 28: 477-487 (1982)

The invention also includes a hybridoma cell line capable of producing such an antibody.

The invention further includes a method for producing an antibody which is capable of binding to sICAM-1 according to claim 1 and not to ICAM-1 comprising the steps of
(a) preparing a peptide-protein conjugate said peptide-protein conjugate specific to at least a portion of the unique 11 amino acid sequence present in sICAM-1 according to claim 1,
(b) immunizing an animal with said peptide-protein conjugate,
(c) boosting the animals, and
(d) obtaining the antisera.

The antibodies would be capable of binding to sICAM-1 according to claim 1 and not capable of binding to ICAM-1. The invention includes the hybridoma cell line which produces an antibody of the same specificity, the antibody produced by the hybridoma cell and the method of production.

This invention further includes a method for substantially reducing the infection of human rhinoviruses of the major receptor group comprising the step of contacting the virus with sICAM-1 or a functional derivative thereof according to claim 1.

### BRIEF DESCRIPTION OF THE DRAWINGS

Figure 1 shows the nucleotide and amino acid sequence of sICAM-1.

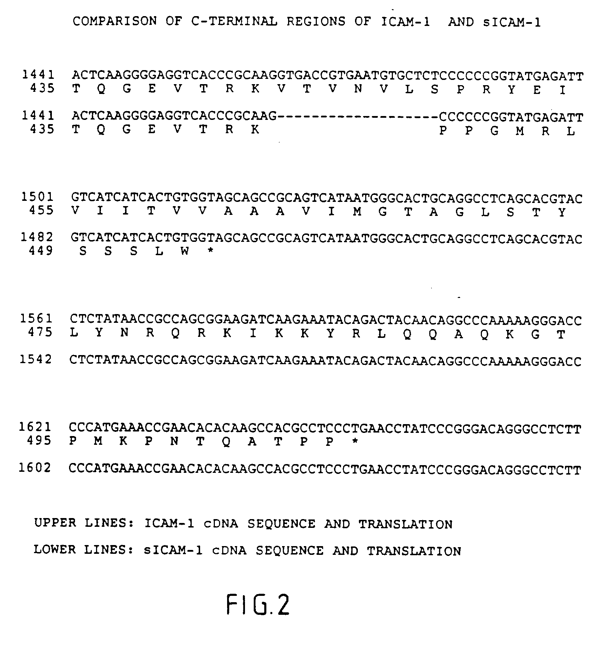

Figure 2 is a comparision of the C-terminal regions of sICAM-1 and ICAM-1. The nucleotide and deduced amino acid sequences of ICAM-1 and sICAM-1 are shown beginning at amino acid residue 435. Dashes in the sICAM-1 sequence indicate missing nucleotides. The positions of the stop codons in both proteins are indicated by an asterisk.

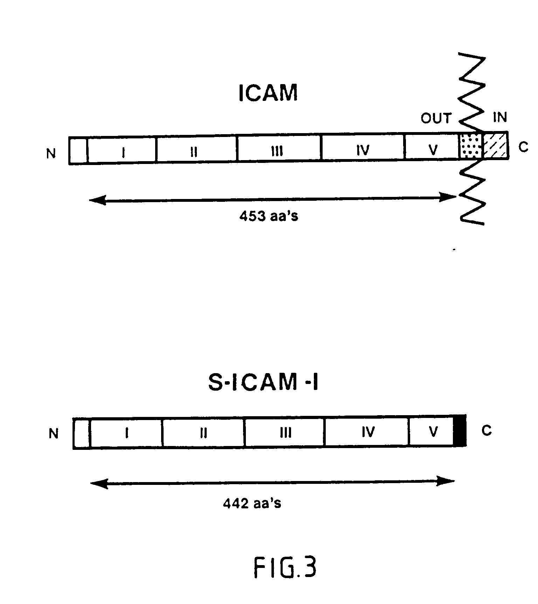

Figure 3 is a comparison of the structure of sICAM-1 and ICAM-1. The membrane spanning region of ICAM-1 is indicated by the stippled box and the cytoplasmic domain by the hatched box. The novel C-terminus of sICAM-1 is indicated by the solid box. The five predicted domains showing homology with immunoglobulin are numbered I to V.

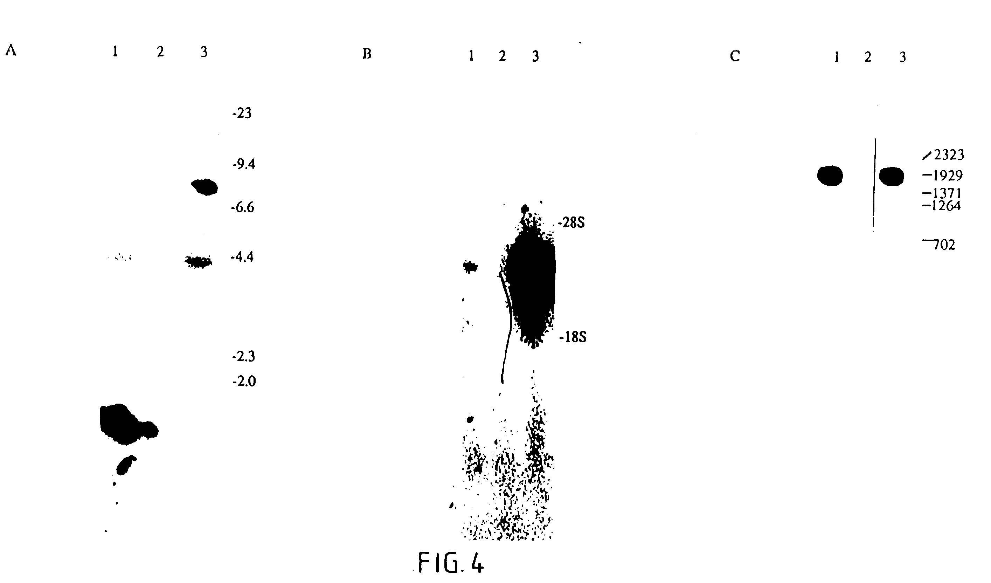

Figure 4 shows the ICAM-1 gene and its expression in HRR transfectants. A: Southern blot of HeLa (Lane 1), LTK- (Lane 2) and HE1 (Lane 3) DNA restricted with Eco R1 and probed with the oligonucleotide ICAM-1;B: Northern blot of HeLa (Lane 1), Lkt⁻(Lane 2), and HE1 (Lane 3). poly A+ RNA probed with the oligonucleotide ICAM-1; C: PCR amplification of cDNA prepared from HeLa (Lane 1), Ltk⁻ (Lane 2) and HE1 (Lane 3) poly A+ RNA. The primers used were from the N-terminal and C-terminal coding regions of ICAM-1 having the sequence ggaattcATGGCTCCCAGCAGCCCCCGGCCC and ggaattcTCAGGGAGGCGTGGCTTGTGTGTT. Upper case denotes ICAM-1 sequence, lower case restriction site linkers. Lanes 1 and 2, 72 hour exposure, Lane 3, 90 minute exposure.

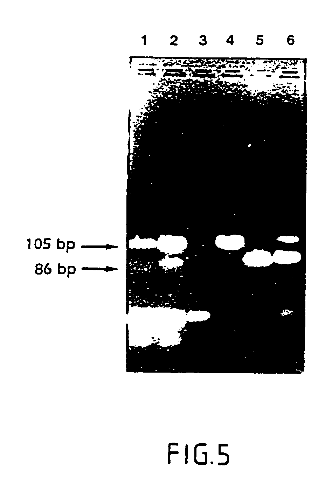

Figure 5 is a gel showing the detection of the ICAM-1 and sICAM-1 mRNAs in HeLa and HE1 cells. PCR amplification was performed on 100ng single stranded CDNA using the primers PCR 5.4 (CTTGAGGGCACCTACCTCTGTCGG) and PCR 3.4 (AGTGATGATGACAATCTCATACCG). Extensions were performed at 72 C for 25 cycles and one tenth of the product was analysed on a 1% agarose/3% NuSieve gel. Lane 1, HeLa cDNA; lane 2, HE1 cDNA; lane 3, LTK⁻ cDNA; lane 4, ICAM-1 phage control;, lane 5, sICAM-1 phage control; lane 6, ICAM-1 + sICAM-1 phage control. Specific amplification products of 105bp and 86bp are indicated by the arrows.

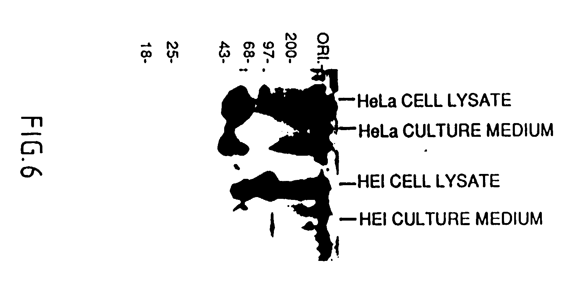

Figure 6 is a Western blot showing the synthesis of a soluble form of ICAM-1 protein by HeLa and HE1 cells. It demonstrates the existence of a protein species in the culture supernatant of HeLa and HE1 cells related to ICAM-1. Equivalent aliquots of cell lysates and culture supernatants were separated by SDS-PAGE, blotted onto nitrocellulose, and probed with a rabbit polyclonal antisera to ICAM-1 followed by ¹⁵I protein A; a species migrating close to the position of membrane-bound ICAM-1 is seen in both HeLa and HE1 culture supernatants.

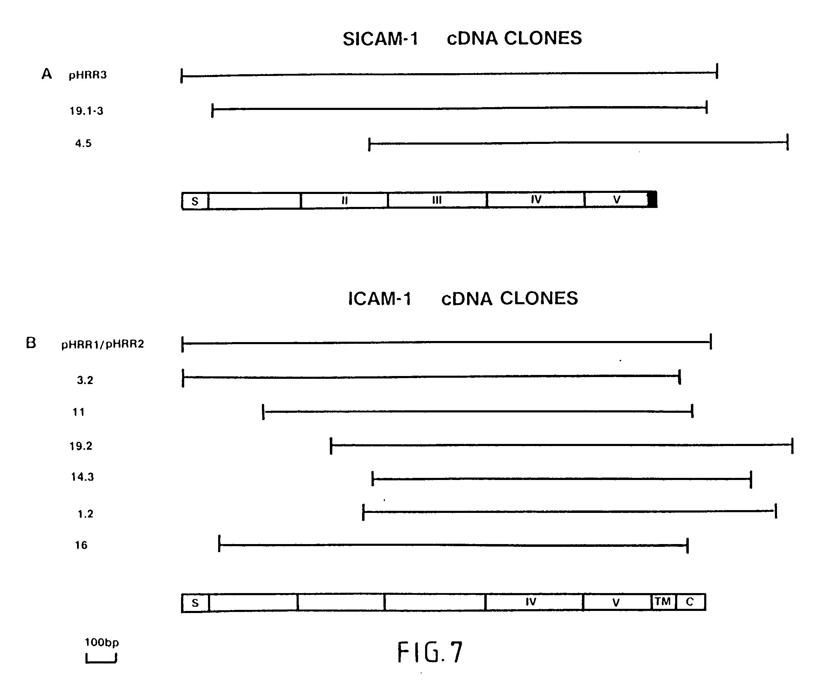

Figure 7 is a graphical representation of the cloned sICAM-1 and ICAM-1 plasmids.

7A. pHRR3 is a full length cDNA encoding sICAM-1 obtained by PCR. Clones 19.1-3 and 4.5 are partial cDNA clones encoding sICAM-1 obtained from an HE1 cDNA library in lambda GT11. Beneath the clones is a schematic of the sICAM-1 molecule. S denotes the signal peptide and I to V the IgG homologous domains. The solid box indicates the unique 11 amino acid C-terminus.

7B. pHRR1 and pHRR2 are full length ICAM-1 cDNA clones obtained by PCR. The remaining ICAM-1 clones were obtained from an HE1 cDNA library in lambda GT11. Beneath the clones is a schematic of the ICAM-1 molecule, showing the signal peptide (S), the five IgG homologous domains (I to V), the transmembrane region (TM) and the cytoplasmic domain (C).

### DESCRIPTION OF THE PREFERRED EMBODIMENTS

One aspect of the present invention relates to the discovery of a soluble natural binding ligand to the receptor binding site of Human Rhinovirus (HRV) and which also binds to LFA-1. This soluble natural molecule is related to but distinct from the molecule designated "Intercellular Adhesion Molecule-1" or "ICAM-1" which is insoluble, bound to the cell membrane and possesses a typical hydrophobic membrane spanning region and a short cytoplasmic tail. The novel protein of the present invention has a DNA sequence which includes a significant difference from the published DNA sequence for ICAM-1. sICAM-1 contains most of the extracellular domain of ICAM-1, which includes the functional domains for multiple functions including HRV and LFA-1 binding, but lacks the membrane spanning and cytoplasmic domains. sICAM-1 retains the ability to bind HRV and LFA-1 and is secreted in a soluble form. The DNA sequence for sICAM-1 contains a deletion of 19 base pairs from nucleotide 1465 to 1483 according to the numbering of Staunton et al, supra (1988). The remainder of the sICAM-1 clone matches the published ICAM-1 sequence with the exception of a substitution of a G for A at nucleotide position 1462 which changes Glu 442 to Lys, as shown in Figure 1. The sequence of amino acid residues in a peptide is designated in accordance with standard nomenclature such as Lehninger's Biochemistry, Worth Publishers, New York, NY (1970). sICAM-1 is a natural product of HeLa and HE1 cells and other human cells which should have the property of binding to and inhibiting the infection of human rhinovirus and Coxsackie A viruses. It also has the property of binding to LFA-1 and may be used to antagonize adhesion of cells mediated by ICAM-1/LFA-1 binding and thus be useful as a therapeutic in treatment of inflammation, graft rejection, suppression of LFA-1 expressing tumor cells and other processes involving cell adhesion. Isolated and purified sICAM-1 protein as a therapeutic would not possess the immunogenic problems associated with foreign proteins. The secretion of a soluble naturally occurring protein eliminates the problems associated with production and purification of an insoluble, cell membrane bound protein, since cell lysis is not required and thus continuous culture can be employed as well as simplified procedures for purification and isolation of sICAM-1.

Non-human mammalian cell lines which express the major human rhinovirus receptor gene have been previously identified and are the subject matter of copending U.S. Patent Application No. 262570 and 262428 filed October 25, 1988, and include references to the ATCC deposits for the cell lines. The major human rhinovirus receptor was identified with monoclonal antibodies which inhibit rhinovirus infection. These monoclonal antibodies recognized a 95 kd cell surface glycoprotein on human cells and on mouse transfectants expressing a rhinovirus-binding phenotype. Purified 95 Kd protein binds to rhinovirus in vitro. Protein sequence from the 95 kd protein showed an identity with that of ICAM-1; a cDNA clone obtained from mouse transfectants expressing the rhinovirus receptor had the same sequence published for ICAM-1, except for the G for A change previously described. Thus it was determined that the major human rhinovirus receptor and ICAM-1 were the same protein. A transfected mouse L-cell line designated HE1 had been isolated which contained and expressed the HRR gene or ICAM-1 gene. The ICAM-1 terminology has been used although it is now recognized that HRR and ICAM-1 are interchangeable.

A randomly primed cDNA library was prepared in lambda GT11 from HE1 polyA+ RNA. The library was screened in duplicate using two oligonucleotides derived from the published sequence of ICAM-1. Oligonucleotide ICAM-1 has the sequence GAGGTGTTCTCAAACAGCTCCAGCCCTTGGGGCCGCAGGTCCAGTTC and oligonucleotide ICAM-3 has the sequence

CGTTGGCAGGACAAAGGTCTGGAGCTGGTAGGGGGCCGAGGTGTTCT.

Eight positive clones were obtained from one screen and three were selected for further study. DNA sequencing of two of the clones showed identity with the published ICAM-1 sequence. The sequence of the third clone, lambda 19.1-3 was significantly different from the other two clones in that there was a deletion of 19 bp from nucleotide 1465 to 1483 according to the numbering of Staunton et al, supra. The 19 bp deletion was present in a second cDNA, lambda HE1-4.5 and independently confirmed using polymerase chain reaction (PCR) generated cDNA. Analysis of the cDNA sequence predicted the existence of a secreted form of ICAM-1 that is generated by an alternative splicing mechanism. Western blot identification of sICAM-1 from culture supernatants of HE1 and HeLa cell lines confirm that the sICAM-1 mRNA sequence encodes a soluble form of ICAM-1 that does not associate with the cell surface but is released into the cell medium. An alternatively spliced mRNA generating a secreted form of another adhesion molecule (NCAM) has been identified (Glower et al, Cell 55:955-964 (1988)), although in NCAM an exon is incorporated into the mRNA while in the present invention an exon is deleted from the mRNA. No alternative mRNA sequence for ICAM-1 had previously been identified. (Staunton et al.)

### sICAM-1 cDNA Clones

A randomly primed cDNA library was constructed in lambda GT11 from HE1 poly A+ by Clontech Laboratories, Palo Alto, CA. The library was screened with two 47 mer oligonucleotide probes from the middle of the ICAM-1 coding sequence. A positive clone designated 19.1-3 was isolated which had an insert of 1.5 kb; a second cDNA clone designated 4.5 which has an insert of 1.25 kb was isolated; and an additional cDNA clone pHRR-3 was obtained by subcloning the products of PCR amplification into Bluescript utilizing the Perkin-Elmer/Cetus DNA Amplification System, Perkin Elmer, Wellesley MA., as shown in figure 4C, lane 3. These clones showed a significant difference from the published ICAM-1 sequence. They all contain a deletion of 19 base pairs from nucleotide 1465 to 1483 according to the numbering of Staunton et al, supra. In order to demonstrate directly that the s-ICAM mRNA is present in HE1 cells and HeLa cels, a PCR experiment was performed using primers which flank the 19bp region which is absent from the s-ICAM mRNA (Figure 8). Using these primers the product from the ICAM-1 mRNA is 105 bp while the s-ICAM-1 product is 19 bp shorter i.e. 86 bp. This experiment shows that both HE1 cells and HeLa cells contain both forms of the ICAM-1 mRNA while the control L-cells do not. A synthetic oligonucleotide designated PCR3.2 having the following sequence: ggaattcTCACTCATACCGGGGGGAGAGCACATT was used to distinguish between cDNA clones containing the 19 bp deletion from clones not containing the 19 bp deletion. The synthetic oligonucleotide does not bind to cDNA clones which contain the 19 bp deletion. In addition, partial sequence of the cDNA 19.1-3 and PHRR-3 confirmed the 19 bp deletion. This data indicates that there are at least two different and distinct ICAM-1 species in HE1 cells. The insoluble ICAM-1 of the prior art and a novel soluble form as described in the present invention.

The sequences of the deleted (sICAM-1) and the nondeleted (ICAM-1) forms of the Intercellular Adhesion Molecule-1 mRNA represented by the cDNA clones are shown in Figure 2. The sequence at the point of deletion is AGGT consistent with an RNA splice junction. The removal of 19 bases from the mRNA shifts the reading frame and causes the two polypeptide sequences to diverge at amio acid residue 443. The deleted form (sICAm-1) contains an additional 11 residues followed by an in-frame termination codon. This molecule thus consists of 453 amino acids as compared to 505 amino acids for the nondeleted form. Beginning with the N-terminus of ICAM-1, sICAM-1 has 442 amino acids in common with ICAM-1. The deleted form (sICAM-1) contains a unique 11 amino acid C-terminus but lacks the membrane spanning (24 amino acids) and cytoplasmic tail 28 amino acids) domains of ICAm-1, as shown in Figure 3.

### ICAM-1 cDNA Clones

A plurality of methods may be used to clone genes. One method is to use two partially overlapping 47mer oligonucleotide probes. These two probes termed oligonucleotide ICAM-1 and oligonucleotide ICAM-3 were synthesized from the published ICAM-1 sequence. The ICAM-1 oligonucleotide was labeled to high specific activity and hybridized to a Southern blot under high stringency conditions. As shown in figure 4A, a single band of 4.4 kb was detected in HeLa, HE1 and two primary HRR transfectant cell lines and was absent from Ltk⁻ cells. This result confirms that the HRR transfectants contain the human ICAM-1 gene. The size of the fragment agrees with Simmons et al but differs from Staunton et al probably reflecting a restriction site polymorphism.

The ICAM-1 oligonucleotide was used to probe a Northern blot of poly A+ RNA from the same cell lines. As shown in figure 4B, an mRNA of 3.3 kb was detected in HeLa, HE1, and primary transfectant cell lines but was absent from Ltk⁻ cells. The signal in HE1 cells was many times stronger than the other cell lines indicating a much higher level of mRNA in HE1 cells. This is in agreement with the higher level of HRR (ICAM-1) expression in HE1 cells. A second 2.4 kb RNA was also detected in HE1 cells. These data confirm that the human ICAM-1 mRNA is expressed in HRR transfectants. See figure 4B.

The human ICAM-1 gene was isolated from the HE1 transfectant using polymerase chain reaction (PCR) amplification utilizing the Perkin-Elmer/Seats DNA Amplification System, Perkin Elmer, Wellesley MA. PCR amplification was performed on single stranded cDNA made from HeLa, Ltd⁻ and HE1 RNA. Primers were made from the 5′ and 3′ coding regions of the published ICAM-1 sequence. ICAM-1 specific amplification products were detected by hybridization of a Southern blot of the PCR reactions using the ICAM-1 oligonucleotide. As shown in figure 4C, a single band of approximately 1600 bp which matches the predicted size was amplified from HeLa cells and HE1 cells but was absent from Ltk⁻ cells. The amplification product was cloned into Bluescript (Strategene, San Diego, CA) and two independent clones designated PHRR1 and PHRR2 were obtained. The complete sequence of PHRR2 showed 100% identity with the published ICAM-1 coding sequence with the exception of a single G to A change previously described.

A lambda GT11 library made from randomly primed HE1 cDNA was screened with the ICAM-1 and ICAM-3 probes and eight positive clones were isolated. Six clones as shown in figure 7 were selected for further study and were anlayzed by partial DNA sequencing. A total of approximately 1000 nucleotides of sequence derived from these clones showed identity with the ICAM-1 sequence.

### Purification and Isolation of Soluble Protein

HeLa and HE1 cells are grown under standard conditions in DMEM (Dulbecco's Modified Essential Media) with 10% Fetal Bovine Serum. Conditioned media from these cells is harvested and centrifuged or filtered to remove cells or cellular debris. The cell-membrane bound ICAM-1 is not present in the supernatant. This media is then absorbed to a monoclonal antibody-sepharose resin (the monoclonal antibody c78.4A being an example) in which the monoclonal antibody is directed to ICAM-1 or sICAM-1 and the unabsorbed proteins are washed from the resin with a physiological saline buffer, such as phosphate-buffered saline. The bound sICAM-1 is then eluted under conditions that preserve the native conformation of the protein, as described in copending application Ser. No. 262428 filed October 25, 1988. The sICAM-1 may be further purified by lectin affinity chromotography, ion exchange chromatography, or gel filtration.

mRNA transcribed in vitro from cDNA encoding sICAM in the Bluescript vector (Strategene) was translated in vitro. In the absence of microsomal membranes, an unglycosylated protein with an apparent MW of 52,000 daltons was obtained; in the presence of microsomal membranes, a glycosylated species of 72,400 daltons was obtained which was sequestered within the microsomal membrane, indicating that the sICAM polypeptide is correctly translocated, processed, and glycosylated by the microsomal membranes.

cDNA's encoding tmICAM and sICAM in the CDM8 vector (See, B. and Aruffo, A. PNAS 84:3365 (1987) were transfected into COS cells and mouse L cells using the DEAE-dextran technique. AT 72 hr, the cells were analyzed by two methods: (1) FACS analysis with anti-ICAM Mab (c78.4) for cell membrane expression of ICAM species and (2) metabolic labeling followed by immunoabsorption with anti-ICAM Mab of cell supernatants and cell lysates. The results from the metabolic labelling indicated intracellular accumulation of a 68,000 dalton species in sICAM-transfected cells but no detectable secretion of sICAM into th supernatant. These data are consistent with sICAM being secreted through the "Regulated" secretory pathway (R. B. Kelly, Science 230:25 (1985)).

Antibody probes specific for sICAM and for ICAM-1 were prepared. The synthetic peptides S-PEP,

P P G M R L S S S L W (C)

derived from a unique 11 amino acid sequence at the C-terminus of sICAM, and P002, derived from the C-terminus of ICAM-1,

G T P M K P N T Q A T P P (C)

was made and purified; the C-terminal C residues in parentheses were added to facilitate coupling of the peptides to protein carriers. The synthetic peptide was coupled to KLH (Keyhole Limpit Hemocyanin) by standard procedures and the conjugate injected into rabbits to product anti-peptide antisera were shown to specifically bind to their respective peptide immunogens.

In connection with the present application the following microorganisms have been deposited with the American Type Culture Collection:
A Mammalian cell line, HE-1 having the ATCC-designation CRL 9592,
a Mammalian cell line, HRVRI (HRRI) having the ATCC-designation CRL 9593 and
a Hybridoma cell line, CIII 92.1A having the ATCC-designation HB 9594.

## Claims (Claims for the following Contracting State(s): AT, BE, CH, LI, DE, DK, FR, GB, IT, LU, NL, SE)

1. Human soluble intercellular adhesion molecule-1, according to the sequence disclosed in Figure 1, or a functional derivative thereof, with the provisio that the 11 C-terminal amino acids disclosed in Figure 1 remain unchanged.

2. An intercellular adhesion molecule obtained from HeLa, HE1, and primary transfectant cells thereof characterized by the following:
(A) soluble in the absence of detergent and
(B) being the translation product of the mRNA sequences as shown in Figure 1

3. A purified and isolated DNA sequence characterized by the DNA sequence encoding human soluble intercellular adhesion molecule-1, as set forth in Figure 1.

4. A host cell containing the DNA sequence of claim 3.

5. A method of recovering soluble intercellular adhesion molecule-1 according to claim 1 in substantially pure form characterized by the steps of:
(A) removing the supternatant from unlysed cells,
(B) introducing the supernatant to an affinity matrix containg immobilized antibody capable of binding to sICAM-1, preferably antibodies against ICAM-1 and sICAM-1, or derivatives thereof,
(C) permitting said sICAM-1 to bind to said antibody of said matrix,
(D) washing said affinity matrix to remove unbound contaminants, and
(E) recovering said slCAM-1 in substantially pure form by eluting said slCAM-1 from said matrix.

6. The method of claim 5 further characterized by introducing the supernatant to a lectin or wheat germ agglutinin column before or after the supernatant is introduced to said antibody column.

7. An antibody preferably monoclonal, capable of binding to slCAM-1 according to claim 1 and not binding to insoluble ICAM-1, preferably capable of binding to the 11 amino acid sequence C terminus as shown in Figure 1.

8. A pharmaceutical composition for reducing the infection of human rhinovirus comprising the protein of claims 1 or 2.

## Claims (Claims for the following Contracting State(s): ES)

1. A method of recovering soluble intercellular adhesion molecule-1 according to the sequence disclosed in Figure 1, or a functional derivative thereof, with the proviso that the 11C-terminal amino acids disclosed in Figure 1 remain unchanged, in substantially pure form characterized by the steps of:
(A) removing the supernatant from unlysed cells,
(B) introducing the supernatant to an affinity matrix containing immobilized antibody capable of binding to ICAM-1 preferably antibodies against sICAM-1 and sICAM-1, or derivatives thereof,
(C) permitting said sICAM-1 to bind to said antibody of said matrix,
(D) washing said affinity matrix to remove unbound contaminants, and
(E) recovering said sICAM-1 in substantially pure form by eluting said sICAM-1 from said matrix.

2. The method of claim 1 further characterized by introducing the supernatant to a lectin or wheat germ agglutinin column before or after the supernatant is introduced to said antibody column.

## Claims (Claims for the following Contracting State(s): GR)

1. A pharmaceutical composition for reducing the infection of human rhinovirus comprising human soluble intercellular adhesion molecule-1 according to the sequence disclosed in Figure 1, or a functional derivative thereof, with the provisio that the 11 C-terminal amino acids disclosed in Figure 1 remain unchanged.

2. A pharmaceutical composition for reducing the infection of human rhinovirus comprising an intercellular adhesion molecule obtained from HeLa, HE1, and primary transfectant cells thereof characterized by the following:
(A) soluble in the absence of detergent and
(B) being the translation product of the mRNA sequences as shown in Figure 1.

3. A method of recovering soluble intercellular adhesion molecule-1 according to claim 1 in substantially pure form characterized by the steps of:
(A) removing the supernatant from unlysed cells,
(B) introducing the supernatant to an affinity matrix containing immobilized antibody capable of binding to sICAM-1, preferably antibodies against ICAM-1 and sICAM-1, or derivatives thereof,
(C) permitting said sICAM-1 to bind to said antibody of said matrix,
(D) washing said affinity matrix to remove unbound contaminants, and
(E) recovering said sICAM-1 in substantially pure form by eluting said sICAM-1 from said matrix.

4. The method of claim 3 further characterized by introducing the supernatant to a lectin or wheat germ agglutinin column before or after the supernatant is introduced to said antibody column.

## Patentansprüche (Patentansprüche für folgende(n) Vertragsstaat(en): AT, BE, CH, LI, DE, DK, FR, GB, IT, LU, NL, SE)

1. Humanes lösliches interzelluläres Adhäsionsmolekül-1 mit der in Fig. 1 offenbarten Sequenz, oder ein funktionales Derivat davon, mit der Maßgabe, daß die in Fig. 1 offenbarten 11 C-terminalen Aminosäuren unverändert bleiben.

2. Interzelluläres Adhäsionsmolekül erhalten aus HeLa-, HE1 - und primären Transfektanten-Zellen davon,
**gekennzeichnet** durch die folgenden Eigenschaften,
(A) löslich bei Fehlen eines Detergens und
(B) Identität mit dem Translationsprodukt der in Fig. 1 gezeigten mRNA-Sequenzen.

3. Gereinigte und isolierte DNA-Sequenz,
**gekennzeichnet** durch die DNA-Sequenz kodierend für humanes lösliches interzelluläres Adhäsionsmolekül-1, wie in Fig. 1 angegeben.

4. Wirtszelle enthaltend die DNA-Sequenz von Anspruch 3.

5. Verfahren zur Gewinnung des löslichen interzellulären Adhäsionsmolekül-1 nach Anspruch 1 in im wesentlichen reiner Form **gekennzeichnet** durch die Schritte:
(A) Entfernen des Überstands aus unlysierten Zellen.
(B) Aufgeben des Überstands auf eine Affinitätsmatrix, enthaltend immobilisierten Antikörper, der zur Bindung an sICAM-1 in der Lage ist, vorzugsweise Antikörper gegen ICAM-1 und sICAM-1 oder Derviate davon,
(C) Ermöglichen, daß das sICAM-1 sich an den Antikörper der Matrix bindet,
(D) Waschen der Affinitätsmatrix unter Entfernung ungebundener Kontaminanten, und
(E) Gewinnen des sICAM-1 in im wesentlicher reiner Form durch Elution des sICAM-1 von der Matrix.

6. Verfahren nach Anspruch 5, das ferner durch Aufgeben des Überstands auf eine Lectin- oder Weizenkeim-Agglutininsäule vor oder nachdem der Überstand auf die Antikörpersäule aufgegeben wird, gekennzeichnet ist.

7. Antikörper, vorzugsweise monoklonal, der zur Bindung an sICAM-1 nach Anspruch 1 in der Lage ist, und der nicht an unlösliches ICAM-1 bindet, der vorzugsweise zur Bindung an die 11 Aminosäuresequenz des C-Terminus, wie in Fig. 1 gezeigt, in der Lage ist.

8. Pharmazeutische Zusammensetzung zur Verminderung der Infektion von humanem Rhinovirus, umfassend das Protein der Ansprüche 1 oder 2.

## Patentansprüche (Patentansprüche für folgende(n) Vertragsstaat(en): ES)

1. Verfahren zur Gewinnung eines löslichen interzellulären Adhäsionsmolekül-1 mit der in Fig. 1 offenbarten Sequenz oder ein funktionales Derivat davon, mit der Maßgabe, daß die in Fig. 1 offenbarten 11 C-terminalen Aminosäuren unverändert bleiben, in im wesentlichen reiner Form, **gekennzeichnet** durch die Schritte:
(A) Entfernen des Überstands aus unlysierten Zellen.
(B) Aufgeben des Überstands auf eine Affinitätsmatrix, enthaltend immobilisierten Antikörper, der zur Bindung an sICAM-1 in der Lage ist, vorzugsweise Antikörper gegen ICAM-1 und sICAM-1 oder Derivate davon,
(C) Ermöglichen, daß das sICAM-1 sich an den Antikörper der Matrix bindet,
(D) Waschen der Affinitätsmatrix unter Entfernung ungebundener Kontaminanten, und
(E) Gewinnen des sICAM-1 in im wesentlicher reiner Form durch Elution des sICAM-1 von der Matrix.

2. Verfahren nach Anspruch 1, das ferner durch Aufgeben des Überstands auf eine Lectin- oder Weizenkeim-Agglutininsäule vor oder nachdem der Überstand auf die Antikörpersäule aufgegeben wird, gekennzeichnet ist.

## Patentansprüche (Patentansprüche für folgende(n) Vertragsstaat(en): GR)

1. Pharmazeutische Zusammensetzung zur Verminderung der Infektion von humanem Rhinovirus, umfassend humanes lösliches interzelluläres Adhäsionsmolekül-1 mit der in Fig. 1 offenbarten Sequenz, oder ein funktionales Derivat davon, mit der Maßgabe, daß die in Fig. 1 offenbarten 11 C-terminalen Aminosäuren unverändert bleiben.

2. Pharmazeutische Zusammensetzung zur Verminderung der Infektion von humanem Rhinovirus, umfassend ein interzelluläres Adhäsionsmolekül, erhalten aus HeLa-, HE1- und primär Transfektanten-Zellen davon, **gekennzeichnet** durch die folgenden Eigenschaften:
(A) löslich bei Fehlen eines Detergens und
(B) Identität mit dem Translationsprodukt der in Fig. 1 gezeigten mRNA-Sequenzen.

3. Verfahren zur Gewinnung eines löslichen interzellulären Adhäsionsmolekül-1 nach Anspruch 1 in im wesentlichen reiner Form, **gekennzeichnet** durch die Schritte:
(A) Entfernen des Überstands aus unlysierten Zellen.
(B) Aufgeben des Überstands auf eine Affinitätsmatrix, enthaltend immobilisierten Antikörper, der zur Bindung an sICAM-1 in der Lage ist, vorzugsweise Antikörper gegen ICAM-1 und sICAM-1 oder Derivate davon,
(C) Ermöglichen, daß das sICAM-1 sich an den Antikörper der Matrix bindet,
(D) Waschen der Affinitätsmatrix unter Entfernung ungebundener Kontaminanten, und
(E) Gewinnen des sICAM-1 in im wesentlicher reiner Form durch Elution des sICAM-1 von der Matrix.

4. Verfahren nach Anspruch 3, das ferner durch Aufgeben des Überstands auf eine Lectin- oder Weizenkeim-Agglutininsäule vor oder nachdem der Überstand auf die Antikörpersäule aufgegeben wird, gekennzeichnet ist.

## Revendications (Revendications pour l'(les) Etat(s) contractant(s) suivant(s): AT, BE, CH, LI, DE, DK, FR, GB, IT, LU, NL, SE)

1. Molécule-1 humaine soluble d'adhésion intercellulaire selon la séquence révélée dans la figure 1, ou un dérivé fonctionnel de celle-ci, avec la condition que les 11 acides aminés du côté C terminal révélés dans la figure 1 restent inchangés.

2. Molécule d'adhésion intercellulaire obtenue à partir de cellules de HeLa, HE1 et de transfectants primaires, caractérisée par les points suivants :
(A) soluble en l'absence de détergent et
(B) étant le produit de traduction des séquences d'ARNm comme montré dans la figure 1.

3. Séquence d'ADN purifiée et isolée, caractérisée par la séquence d'ADN codant pour la molécule-1 humaine soluble d'adhésion intercellulaire, comme présenté dans la figure 1.

4. Cellule hôte contenant la séquence d'ADN de la revendication 3.

5. Procédé de récupération de la molécule-1 soluble d'adhésion intercellulaire selon la revendication 1 sous une forme en substance pure, caractérisé par les étapes de :
(A) l'élimination du surnageant des cellules non lysées,
(B) l'introduction du surnageant dans une matrice d'affinité contenant un anticorps immobilisé capable de liaison à sICAM-1, de préférence des anticorps contre ICAM-1 et sICAM-1, ou des dérivés de ceux-ci,
(C) la possibilité laissée à ladite sICAM-1 de se lier audit anticorps de ladite matrice,
(D) le lavage de ladite matrice d'affinité pour éliminer les contaminants non liés, et
(E) la récupération de ladite sICAM-1 sous une forme en substance pure par élution de ladite sICAM-1 de ladite matrice.

6. Procédé selon la revendication 5, caractérisé en outre par l'introduction du surnageant dans une colonne de lectine ou d'agglutinine du germe de blé avant ou après l'introduction du surnageant dans ladite colonne d'anticorps.

7. Anticorps, de préférence monoclonal, capable de liaison à sICAM-1 selon la revendication 1 et de non liaison à ICAM-1 insoluble, de préférence capable de liaison au C terminal de la séquence des 11 acides aminés comme montré dans la figure 1.

8. Composition pharmaceutique pour la réduction de l'infection par rhinovirus humain comprenant la protéine de la revendication 1 ou 2.

## Revendications (Revendications pour l'(les) Etat(s) contractant(s) suivant(s): ES)

1. Procédé de récupération de la molécule-1 soluble d'adhésion intercellulaire selon la séquence révélée dans la figure 1, ou un dérivé fonctionnel de celle-ci, avec la condition que les 11 acides aminés du côté C terminal révélés dans la figure 1 restent inchangés, sous une forme en subtance pure, caractérisé par les étapes de :
(A) l'élimination du surnageant des cellules non lysées,
(B) l'introduction du surnageant dans une matrice d'affinité contenant un anticorps immobilisé capable de liaison à sICAM-1, de préférence des anticorps contre ICAM-1 et sICAM-1, ou des dérivés de ceux-ci,
(C) la possibilité laissée à ladite sICAM-1 de se lier audit anticorps de ladite matrice,
(D) le lavage de ladite matrice d'affinité pour éliminer les contaminants non liés, et
(E) la récupération de ladite sICAM-1 sous une forme en substance pure par élution de ladite sICAM-1 de ladite matrice.

2. Procédé selon la revendication 1, caractérisé en outre par l'introduction du surnageant sur une colonne de lectine ou d'agglutinine du germe de blé avant ou après l'introduction du surnageant à ladite colonne d'anticorps.

## Revendications (Revendications pour l'(les) Etat(s) contractant(s) suivant(s): GR)

1. Composition pharmaceutique pour la réduction de l'infection par le rhinovirus humain, comprenant la molécule-1 humaine soluble d'adhésion intercellulaire selon la séquence révélée dans la figure 1, ou un dérivé fonctionnel de celle-ci, avec la condition que les 11 acides aminés du côté C terminal dans la figure 1 restent inchangés.

2. Composition pharmaceutique pour la réduction de l'infection par le rhinovirus humain comprenant une molécule d'adhésion intercellulaire obtenue à partir de cellules de HeLa, HE1 et de transfectants primaires, caractérisée par les points suivants :
(A) soluble en l'absence de détergent et
(B) étant le produit de traduction des séquences d'ARNm comme montré dans la figure 1.

3. Procédé de récupération de la molécule-1 soluble d'adhésion intercellulaire selon la revendication 1 sous une forme en substance pure, caractérisé par les étapes de :
(A) l'élimination du surnageant des cellules non lysées,
(B) l'introduction du surnageant dans une matrice d'affinité contenant un anticorps immobilisé capable de liaison à sICAM-1, de préférence des anticorps contre ICAM-1 et sICAM-1, ou des dérivés de ceux-ci,
(C) la possibilité laissée à ladite sICAM-1 de se lier audit anticorps de ladite matrice,
(D) le lavage de ladite matrice d'affinité pour éliminer les contaminants non liés, et
(E) la récupération de ladite sICAM-1 sous une forme en substance pure par élution de ladite sICAM-1 de ladite matrice.

4. Procédé selon la revendication 3 en outre caractérisé par l'introduction du surnageant sur une colonne de lectine ou d'agglutinine du germe de blé avant ou après l'introduction du surnageant à ladite colonne d'anticorps.
